**Europäisches Patentamt**

**European Patent Office**

**Office européen des brevets**

(11) Veröffentlichungsnummer: **0 251 112**
**A2**

(12) # EUROPÄISCHE PATENTANMELDUNG

(21) Anmeldenummer: **87108940.5**

(22) Anmeldetag: **23.06.87**

(51) Int. Cl.4: **C07C 67/36** , **C07C 69/06**

(30) Priorität: **26.06.86 DE 3621362**

(43) Veröffentlichungstag der Anmeldung:
**07.01.88 Patentblatt 88/01**

(84) Benannte Vertragsstaaten:
**BE DE ES FR GB IT NL**

(71) Anmelder: **BASF Aktiengesellschaft**
**Carl-Bosch-Strasse 38**
**D-6700 Ludwigshafen(DE)**

(72) Erfinder: **Stroefer, Eckhard, Dr.**
**Werderstrasse 20**
**D-6800 Mannheim 1(DE)**
Erfinder: **Aders, Wolf-Karlo, Dr.**
**Haardtstrasse 40**
**D-6701 Ellerstadt(DE)**
Erfinder: **Keller, Peter, Dr.**
**Brunnengasse 24**
**D-6940 Weinheim(DE)**
Erfinder: **Rotermund, Gerhard W., Dr.**
**Gluckstrasse 5**
**D-6800 Mannheim 1(DE)**
Erfinder: **Mueller, Franz-Josef, Dr.**
**Mueller-Thurgau-Weg 1**
**D-6706 Wachenheim(DE)**
Erfinder: **Steiner, Wolfgang, Dr.**
**Hauptstrasse 38**
**D-6701 Friedelsheim(DE)**

(54) **Verfahren zur Herstellung von Alkylformiaten.**

(57) Herstellung von $C_1$-$C_6$-Alkylformiaten durch Umsetzung eines Alkanols mit Kohlenmonoxid in Gegenwart eines Alkali-oder Erdalkalialkoholates bei erhöhter Temperatur und einem Druck von 20 bis 150 bar, ggf. in Gegenwart eines aprotischen Lösungsmittels, wobei man dem Reaktionsgemisch 20 bis 70 Gew.% des entstehenden Alkylformiates, bezogen auf das Alkanol, zusetzt.

EP 0 251 112 A2

## Verfahren zur Herstellung von Alkylformiaten

Die vorliegende Erfindung betrifft ein verbessertes Verfahren zur Herstellung von $C_1$-$C_6$-Alkylformiaten durch Umsetzung von Alkoholen mit Kohlenmonoxid in Gegenwart eines Alkali-oder Erdalkalialkoholates bei erhöhter Temperatur und einem Druck von 20 bis 150 bar, ggf. in Gegenwart eines aprotischen Lösungsmittels.

Die Herstellung von Alkylformiaten aus Alkanolen und Kohlenmonoxid ist allgemein z.B. aus Ullmanns Encyklopädie der techn. Chemie, 3. Aufl. (1953), Bd. 3, S. 450-451 bekannt. Da es sich um eine Gleichgewichtsreaktion handelt, die mit zunehmendem CO-Partialdruck auf der Seite des Produkts liegt und die Reaktionsgeschwindigkeit mit dem CO-Druck ansteigt, wird großtechnisch bevorzugt im Hochdruckbereich oberhalb von 200 bar umgesetzt. Versuche, Alkylformiate im Mitteldruckbereich, d.h. im Bereich von 20 bis 150 bar herzustellen und die dadurch bedingte niedrigere Reaktionsgeschwindigkeit durch Erhöhung der Katalysatormenge zu kompensieren verlaufen unbefriedigend, da es zu Salzabscheidungen und Verkrustungen in den Apparaturen kommt.

Um das Problem der Salzabscheidungen zu umgehen, wurden dem Reaktionsgemisch gemäß der DE-OS 30 37 089 nicht-ionische Komplexbildner z.B. solche mit einem Polyethylenglykol-Strukturelement zugesetzt.

Aus der GB-PS 1 511 961 geht hervor, daß Salzablagerungen bei der Methylformiatsynthese im Mitteldruckbereich von 20 bis 110 bar durch intensive Zirkulation des Reaktionsgemisches vermieden werden können. Diese Schrift lehrt weiterhin, daß es im allgemeinen nicht vorteilhaft ist, die Umwandlung von Methanol weiter als 70 % der maximal möglichen Gleichgewichtsumwandlung fortzusetzen, da die Reaktionsgeschwindigkeit dann stark abfällt (s. auch A. Agullo, T. Hortenko in Hydrocarbon Processing, Nov. 1980, S. 120 bis 126). Die Raum-Zeit-Ausbeute des beschriebenen Verfahrens liegt bei maximal 5, 16 kg Methylformiat pro Stunde und m³.

Es ist aus der DE-OS 14 93 058 ferner bekannt, daß durch Zusatz von Lösungsmitteln wie Kohlenwasserstoffen, Ethern und/oder Estern die Umsetzungsgeschwindigkeit bei der Methylformiatherstellung erhöht wird. Das Lösungsmittel wird vorzugsweise in etwa stöchiometrischem Verhältnis, bezogen auf Methanol, eingesetzt. Kriterium für die Geschwindigkeitserhöhung ist die Anfangsbildungsgeschwindigkeit des Methylformiates. Als besonders vorteilhaftes Lösungsmittel wird Dioxan aufgeführt. Die Raum-Zeit-Ausbeute dieses Verfahrens ist nicht befriedigend und beträgt maximal 200 kg Methylformiat pro Stunde und m³.

Der Erfindung lag nun die Aufgabe zugrunde, ein Verfahren zur Herstellung von Alkylformiaten im Mitteldruckbereich von 20 bis 150 bar zu finden, bei dem in Gegenwart geringer Katalysatorkonzentrationen hohe Reaktionsgeschwindigkeiten und Umsätze bei einfacher und billiger Betriebsweise erzielt werden können. Neben einer hohen Raum-Zeit-Ausbeute sollte aufgabengemäß eine Ablagerung des Katalysators sowie dessen Desaktivierung durch Akali-bzw. Erdalkaliformiatbildung weitgehend vermieden werden.

Demgemäß wurde ein Verfahren zur Herstellung von $C_1$-$C_6$-Alkylformiaten durch Umsetzung eines Alkanols mit Kohlenmonoxid in Gegenwart eines Alkali-oder Erdalkalialkoholates bei erhöhter Temperatur und einem Druck von 20 bis 150 var, gegebenenfalls in Gegenwart eines aprotischen Lösungsmittels, gefunden, das dudurch gekennzeichnet ist, daß man dem Reaktionsgemisch 20 bis 70 Gew.% des entstehenden Alkylformiates, bezogen auf das Alkanol, zusetzt.

Dem erfindungsgemäßen Verfahren liegt die Beobachtung zugrunde, daß die maximale Umsetzungsgeschwindigkeit bei der Umsetzung von Alkanolen mit 1 bis 6 Kohlenstoffatomen, mit Kohlenmonoxid im Mitteldruckbereich nicht zu Beginn der Umsetzung erreicht wird, sondern kurz vor dem Ende, also dem Erreichen des Gleichgewichtszustandes. Demnach wird die Reaktion zur Erzielung maximaler Raum-Zeit-Ausbeuten erfindungsgemäß so durchgeführt, daß stets eine hohe Konzentration an Alkylformiat im Reaktionsgemisch vorliegt. Dem Reaktionsgemisch wird soviel des entstehenden Alkylformiates zugesetzt, daß die Konzentration etwa 20 bis 70, insbesondere 30 bis 50, vorzugsweise 30 bis 40 Gew.%, bezogen auf das eingesetzte Alkanol, beträgt.

Eine weitere Erhöhung der maximalen Umsetzungsgeschwindigkeit läßt sich dadurch erreichen, daß man die Umsetzung in Gegenwart von inerten, aprotischen Lösungsmitteln wie aliphatischen, cycloaliphatischen, araliphatischen oder aromatischen Kohlenwasserstoffen, durch polare Gruppen wie $C_1$-$C_4$-Alkoxy-, Halogen oder Nitrogruppen substituiertem Benzol, halogenierten Kohlenwasserstoffen, Ethern, insbesondere höheren Ethern wie Ethylenglykolethern oder Diethylenglykolethern, Nitrilen, N,N-Dialkylamiden, Sulfoxiden, Ketonen oder 5-oder 6-gliedrigen heterocyclischen Verbindungen, die 1 oder 2 Heteroatome wie Stickstoff oder Sauerstoff enthalten und mit einem Benzolring kondensiert sein können, durchführt.

Beispielweise seien folgende Verbindungen aufgeführt: Octan, Cyclohexan, Xylol, Toluol, Chloroform, Methylenbromid, Nitrobenzol, Chlorbenzol, Dichlorbenzol, tert.-Butylmethylether, Ethylenglykoldimethylether, Diethylenglykoldiethylether, Acetonitril, Propionitril, Benzonitril, N,N-Dimethylformamid, Hexamethlyphosphorsäuretriamid, Dimethylsulfoxid, Diethylketon, Methylethylketon, Hexanon-(3), Acetophenon, Benzophenon sowie als Heterocyclen Furan, Tetrahydrofuran, Benzofuran, 1,4-Dioxan, 1,3-Dioxolan, Thiophen, N-Methylpyrrol, N-Methylmorpholin, N-Ethylpiperidin, Pyrazin, Pyrimidin, Chinolin, Isochinolin und insbesondere Pyridin.

Vorteilhaft können polare Lösungsmittel mit einer Donatorzahl größer als 15, insbesondere 20 bis 40, z.B. Pyridin verwendet werden. Es zeigte sich, daß die Reaktionsgeschwindigkeit mit zunehmender Donatorzahl (DN) des Lösungsmittels ansteigt (zur Bedeutung und Bestimmung der Donatorzahl siehe C. Reichardt, Solvent Effects in Organic Chemistry, 1979). So besteht z.B. im Fall der Methylformiatsynthese folgendes Verhältnis zwischen den Donatorzahlen der Lösungsmittel und den gemessenen maximalen Umsetzungsgeschwindigkeiten, bezogen auf Toluol:Toluol (DN = 4,8):Dioxan (DN = 14,8):Pyridin (DN = 33,8):Hexamethylphosphorsäuretriamid (DN = 38) = 1:1,7:2,9:3,1. Eine Ausnahme bilden Carbonsäureester, die trotz relativ niedriger Donatorzahl hohe Geschwindigkeiten bewirken. So liegt die maximale Umsetzungsgeschwindigkeit für Methylacetat (DN = 11,0) bei 2,2, bezogen auf Toluol. Überraschenderweise sind Carbonsäureester mit 2 bis 20, insbesondere 2 bis 10 Kohlenstoffatomen besonders vorteilhafte Lösungsmittel, wobei Acetate, insbesondere $C_1$-$C_6$-Alkylacetate, bevorzugt sind. Hier seien z.B. die Ester der Essigsäure, Propionsäure, Buttersäure, Valeriansäure, Capronsäure, Cyclohexancarbonsäure oder Benzoesäure mit Alkoholen wie Methanol, Ethanol, Propanol, Butanal, Pentanol oder Hexanol genannt. Auch können entsprechende Ester von Dicarbonsäuren wie Oxalsäure, Malonsäure, Bernsteinsäure oder Adipinsäure verwendet werden.

Zweckmäßigerweise werden mit dem eingesetzten Alkanol mischbare Lösungsmittel verwendet, deren Siedepunkte höher liegen als die der gebildeten Formiate. Dies bringt den Vorteil mit sich, daß die Produkte leicht destillativ aus dem Reaktionsgemisch abgetrennt werden können und die verbleibenden Gemische nach Ersatz von Alkanol und ggf. Katalysatorverlusten wieder in die Synthese zurückgeführt werden können.

Die Lösungsmittelmenge ist nicht besonders kritisch, es können stöchiometrische oder mehr als stöchiometrische Mengen, bezogen auf den eingesetzten Alkohol, verwendet werden. Vorzugsweise wird die Umsetzung jedoch in Gegenwart von 20 bis 70, insbesondere 40 bis 60 Gew.%, bezogen auf den eingesetzten Alkohol, vorgenommen. Durch die Zugabe des Lösungsmittels tritt zwar ein Verlust an Reaktionsraum ein, jedoch steigt dennoch die Raum-Zeit-Ausbeute an, da die Reaktionsgeschwindigkeit stark erhöht wird. Gleichzeitig wird das thermodynamische Gleichgewicht zugunsten des Alkylformiats verschoben.

Nach dem erfindungsgemäßen Verfahren werden Alkanole mit 1 bis 6 Kohlenstoffatomen, z.B. Methanol, Ethanol, Propanol, Isopropanol, Butanol, Pentanol oder Hexanol mit Kohlenmonoxid umgesetzt. Besonders vorteilhaft ist das Verfahren zur Herstellung von Methylformiat geeignet.

Die Umsetzung der Alkanole mit Kohlenmonoxid erfolgt, abgesehen von dem erfindungsgemäßen Formiatzusatz, in bekannter Weise, z.B. diskontinuierlich oder vorzugsweise kontinuierlich bei guter Durchmischung des Reaktionsmediums und Temperaturen von 30 bis 120, insbesondere 60 bis 80°C nach den dafür üblichen Techniken. Als Reaktoren dienen beispielsweise Rührkessel, Strahldüsenreaktoren, Blasensäulen oder eine Kaskade solcher Apparate. Bei kontinuierlichem Betrieb wird der Reaktor vorteilhaft so betrieben, daß die stationäre Reaktionskonzentration den Bedingungen der maximalen Reaktionsgeschwindigkeiten im diskontinuierlichen Versuch entsprechen. Die Isolierung der Alkylformiate wird vorteilhaft destillativ vorgenommen.

Als Katalysatoren können Alkoholate der Erdalkalimetalle, z.B. Calcium, Strontium oder Barium und insbesondere Alkalimetallalkoholate wie beispielsweise Lithium-, Natrium-oder Kaliumalkoholat verwendet werden. Aus Wirtschaftlichkeitsgründen ist die Verwendung von Natriumalkoholaten in der Regel besonders bevorzugt. Prinzipiell sind Alkoholate beliebiger Alkohole geeignet, jedoch wird man im allgemeinen das Alkoholat des systemeigenen Alkohols einsetzen.

Die Umsetzung kann vorteilhaft in Gegenwart von Katalysatormengen unterhalb von 4 Gew.%, bezogen auf das Reaktionsgemisch, durchgeführt werden. Alkoholatgehalte im Konzentrationsbereich von 0,05 bis 4, insbesondere 0,1 bis 2,5 Gew.%, bezogen auf das Reationsgemisch, sind im allgemeinen ausreichend.

Beispiele 1 bis 4

Diskontinuierliche Herstellung von Methylformiat

Die Umsetzung von Methanol (600 ml, 14,8 mol in Versuch 1) mit Kohlenmonoxid erfolgte bei einer Temperatur von 60°C in Gegenwart von x Gex.% Natriummethylat, bezogen auf Methanol. Während der Reaktion wurde der CO-Druck über ein Regelsystem und einen Massenflußmesser konstant bei 50 bar gehalten. Die Anzeige des Massenflußmessers ist proportional der Reaktionsgeschwindigkeit (RG). Die Versuchsergebnisse hinsichtlich Reaktionszeit, maximaler Reaktionsgeschwindigkeit und Anteil an Methylformiat im thermodynamischen Gleichgewicht sind Tabelle 1 zu entnehmen.

Tabelle 1

Versuchsergebnisse der Beispiele 1 (ohne Lösungsmittelzusatz)

2 (gewichtsmäßig die Hälfte des Methanols in Beispiel 1 ersetzt durch Pyridin)

3 (analog 2, weniger Katalysator)

4 (analog 1, gewichtsmäßig 30 % des Methanols durch Methylformiat ersetzt)

| Beispiel | NaOCH$_3$ [Gew.%] | Dauer [min] | RG$_{max}$ $= \dfrac{d[CO]}{dt}$ [mol·ml$^{-1}$·min$^{-1}$] | RG$_{max}$ erreicht nach t = [min] | HCOOCH$_3$-Anteil im Reaktions-austrag [Gew.%] a) |
|---|---|---|---|---|---|
| 1 | 1,6 | 420 | ~ 6·10$^{-5}$ | 270 | 78 |
| 2 | 1,6 | 45 | ~ 4·10$^{-4}$ | 30 | 89 |
| 3 | 0,8 | 80 | ~ 1,5·10$^{-4}$ | 50 | 87 |
| 4 | 1,6 | 170 | ~ 6·10$^{-5}$ | 110 | 78 |

a) bezogen auf Methanol

Die Raum-Zeit-Ausbeute liegt bei Versuch 2 um ca. den Faktor 5, bei Versuch 3 um ca. Faktor 2,5 und bei Versuch 4 um den Faktor 1,5 höher als im Vergleichsversuch 1.

Beispiele 4 und 5

Kontinuierliche Herstellung von Methylformiat

Die Umsetzung von Methanol mit Kohlenmonoxid erfolgte in einem Strahldüsenreaktor bei 80°C und 40 bar Kohlenmonoxiddruck in Gegenwart von 2 Gew.% NaOCH$_3$, bezogen auf Methanol. Die Ergebnisse sind in Tabelle 2 aufgeführt.

Tabelle 2

Versuchsergebnisse bei kontinuierlicher Methylformiatherstellung
(T = 80°C, p = 40 bar)

| | Beispiel 5 | Vergleichs-beispiel 6 |
|---|---|---|
| Fremdlösungsmittel $H_3COOCH_3$ (Gew.%, bez. $CH_3OH$) | 50 | - |
| Zupumpgeschwindigkeit des Gemisches (l/h) | 35 | 10 |
| Verweilzeit im Reaktor (h) | 0,6 | 2 |
| $HCOOCH_3$-Anteil im Reaktionsaustrag (Gew.%, bez. Rkt.Gemisch) | 35 | 37 |
| Raum-Zeit-Ausbeute (kg/$m^3 \cdot$h) | 500 | 150 |

## Ansprüche

1. Verfahren zur Herstellung von $C_1$-$C_6$-Alkylformiaten durch Umsetzung eines Alkanols mit Kohlenmonoxid in Gegenwart eines Alkali-oder Erdalkalialkoholates bei erhöhter Temperatur und einem Druck von 20 bis 150 bar, gegebenenfalls in Gegenwart eines aprotischen Lösungsmittels, dadurch gekennzeichnet, daß man dem Reaktionsgemisch 20 bis 70 Gew.% des entstehenden Alkylformiates, bezogen auf das Alkanol, zusetzt.

2. Verfahren nach Anspruch 1, dadurch gekennzeichnet, daß man mit dem eingesetzten Alkanol mischbare Lösungsmittel verwendet, deren Siedepunkte höher liegen als die der gebildeten Formiate.

3. Verfahren nach den Ansprüchen 1 und 2, dadurch gekennzeichnet, daß man als Lösungsmittel Carbonsäureester oder Verbindungen mit einer Donatorzahl größer als 20 verwendet.

4. Verfahren nach den Ansprüchen 1 bis 3, dadurch gekennzeichnet, daß man als Lösungsmittel Acetate verwendet.

5. Verfahren nach den Ansprüchen 1 bis 3, dadurch gekennzeichnet, daß man als Lösungsmittel Pyridin verwendet.

6. Verfahren nach den Ansprüchen 1 bis 5, dadurch gekennzeichnet , daß man das Reaktionsgemisch nach Abtrennung der gebildeten Formiate für die Umsetzung wiederverwendet.

7. Verfahren nach den Ansprüchen 1 bis 6, dadurch gekennzeichnet, daß man die Umsetzung kontinuierlich durchführt.

8. Verfahren nach den Ansprüchen 1 bis 7, dadurch gekennzeichnet, daß man die Umsetzung bei 30 bis 120°C vornimmt.

9. Verfahren nach den Ansprüchen 1 bis 8, dadurch gekennzeichnet, daß man 0,05 bis 4 Gew.% des Katalysators, bezogen auf das Reaktionsgemisch, verwendet.